# EUROPÄISCHE PATENTSCHRIFT

(11) **EP 3 478 660 B1**
(45) Veröffentlichungstag und Bekanntmachung des Hinweises auf die Patenterteilung: **11.08.2021**
(21) Anmeldenummer: 17730829.3
(22) Anmeldetag: 22.06.2017
(51) Int. Cl.: C07D 215/20, B01D 3/14

(54) **VERFAHREN ZUR AUFREINIGUNG VON ETHOXYQUIN**
METHOD FOR PURIFYING ETHOXYQUIN
PROCÉDÉ DE NETTOYAGE D'ÉTHOXYQUINE

(30) Priorität: 29.06.2016 EP 16176852; 28.09.2016 EP 16191008
(43) Veröffentlichungstag der Anmeldung: 08.05.2019
(73) Patentinhaber: BASF SE, 67056 Ludwigshafen am Rhein (DE)
(72) Erfinder: CHATURVEDULA, Sumana, 68623 Lampertheim (DE); KELLER, Andreas, 67056 Ludwigshafen (DE); SIEGEL, Wolfgang, 67056 Ludwigshafen (DE); JOEDECKE, Michael, 67056 Ludwigshafen (DE); HAUBNER, Martin, 67056 Ludwigshafen (DE)
(74) Vertreter: BASF IP Association
(86) Internationale Anmeldenummer: PCT/EP2017/065414
(87) Internationale Veröffentlichungsnummer: WO 2018/001862

(56) Entgegenhaltungen:
- WO-A1-2010/031790
- WO-A1-2017/184498
- CN-A- 105 085 393
- JP-A- S4 935 387
- LESTAK ET AL: "ADVANCED DISTILLATION SAVESE", CHEMICAL ENGINEERING, ACCESS INTELLIGENCE ASSOCIATION, ROCKVILLE, MA, US, Bd. 7, 1. Juli 1997 (1997-07-01), Seiten 72-76, XP001156299, ISSN: 0009-2460

## Beschreibung

Die vorliegende Erfindung betrifft ein Verfahren zur Aufreinigung von Ethoxyquin durch Destillation.

Ethoxyquin als solches ist bekannt, ebenso dessen Aufreinigung mittels verschiedener technischen Grundoperationen. Allerdings war es bisher nicht möglich, hochreines Ethoxyquin zu erhalten, und insbesondere nicht ein solches Ethoxyquin, das weniger als 100 ppm, bevorzugt weniger als 50 ppm, besonders bevorzugt weniger als 10 ppm und ganz besonders bevorzugt weniger als 5 ppm wie beispielsweise weniger als 1 ppm oder gar weniger als 0,5 ppm p-Phenitidin enthält sowie alle Werte dazwischen, beispielsweise weniger als 90, 80, 70, 60, 45, 40, 35, 30, 25, 20, 15, 9, 8, 7, 6, 4, 3, 2, 0,9, 0,8, 0,7, 0,6, 0,4, 0,3, 0,2 oder weniger als 0,1.

Da p-Phenitidin als eine als toxikologisch bedenkliche Substanz bekannt ist, ist eine weitestgehend mögliche Abreicherung davon im Ethoxyquin aber dringend gewünscht. Bisherige Aufreinigungsverfahren schafften es jedoch nicht, Ethoxyquin mit einem p-Phenitidin-Gehalt von weniger als 0,1 Prozent (entsprechend 1000 ppm) bereitzustellen.

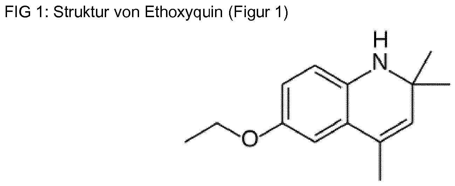

Ethoxyquin (Figur 1) ist der Trivialname für 1,2-Dihydro-6-ethoxy-2,2,4-trimethylchinolin und trägt die CAS-Nummer 91-53-2 sowie die E-Nummer 324.

Ethoxyquin wird aufgrund seiner bekannten antioxidativen Wirkung seit Jahrzehnten im Wesentlichen zur Futtermittelkonservierung eingesetzt, da es unter anderem die Oxidation von Fetten und Vitaminen verhindert oder wenigstens verzögert.

Ethoxyquin kann beispielsweise ausgehend von p-Chlornitrobenzol und Natriumethanolat, Hydrieriung des resultieren Reaktionsproduktes zu p-Phenetidin und anschließende Reaktion mit Aceton zu Ethoxyquin erhalten werden (siehe etwa Thomas A. Unger: Pesticide Synthesis Handbook. William Andrew, 1996, S. 586)

P-Phenitidin kommt als bekannte Verunreinigung in Ethoxyquin vor, wenn es über die Syntheseroute via p-Phenitidin hergestellt wurde. Daher war es ein Bestreben, diese Nebenkomponente aus dem Produkt Ethoxyquin abzureichern.

P-Phenitidin hat einen Schmelzpunkt von 3 °C und einen Siedepunkt von 254 °C. Ethoxyquin hat einen Schmelzpunkt von unter Null °C und einen Siedepunkt von etwa 123 bis 125 °C bei 2,67 hPa.

Übliche Methoden zur Trennung zweier organischer Substanzen sind beispielsweise in "Separation Processes, Introduction", C.Judson King, in Ullmann's Encyclopedia of Industrial Chemistry, 2012, beschrieben. Daraus ist ersichtlich, dass ein Fachmann, vor die Aufgabe gestellt, Ethoxyquin zu reinigen, keine Destillation als großtechnisches Verfahren angewendet hätte.

Vielmehr wir dem Fachmann dort mitgeteilt, dass bei einem Problem der Trennung kleiner Verunreingungen aus einem organischen Produkt (also eine "Feinreinigung") insbesondere adsorptive Prozesse, Ausfällungen/Waschungen mit Säure, Chromatografie-Verfahren, Strippung oder Kristallisation die vielversprechendsten Grundoperationen sein sollten. Destillation wird dagegen laut Ullmann für eine grobe Aufreinigung eingesetzt, also die Abreichung einer Komponente, bevor der Produktstrom dann zu einer Feinreinigung wie zuvor ausgeführt zugeführt wird. Eine Destillation ist bei einem solchen Problem der Feinreinigung von Ethoxyquin dagegen laut Ullmann kein empfehlenswerter Prozessschritt.

Ping He und Robert G.Ackmann, Journal of Agricultural and Food Chemistry, Volume 48, Nummer 8, August 2000, Seiten 3069 bis 3071, "Purification of Ethoxyquin and its two Oxidation Products", beschreiben verschiedene Aufreinigungsmethoden von Ethoxyquin und dem Ethoxyquin-Dimer und einer weiteren Substanz. Ethoxyquin wurde dabei destillativ im Vakuum auf etwa 90 Prozent (Flächenprozent) Reinheit angereichert und danach mittels Kolonnen-Chromatografie auf größer 99 Prozent Reinheit aufkonzentriert. Eine Aufreinigung zu Ethoxyquin mit weniger als 500 ppm p-Phenitidin ist nicht offenbart, weder mittels Destillation noch durch andere Methoden.

Es ist keine Patentliteratur bekannt, die die Bereitstellung von Ethoxyquin zu einer Reinheit von weniger als 1000 ppm p-Phenitidin offenbart.

Bisher nicht bekannt ist daher ein Verfahren zur Bereitstellung von Ethoxyquin in sehr hoher Reinheit mit weniger als 100 ppm p-Phenitidin, insbesondere kein großtechnisches Verfahren. Insbesondere ist auch kein kommerzielles großtechnisches Produkt bekannt, das diese Anforderungen erfüllen würde.

Aufgabe der vorliegenden Erfindung war es daher, ein Ethoxyquin herzustellen, dessen p-Phenitidin-Gehalt weniger als 100 ppm und insbesondere so niedrig wie möglich liegen sollte, um allen toxikologischen Bedenken bei der Anwendung von Ethoxyquin in Bezug auf dessen Verunreinigung mit p-Phenitidin Rechnung zu tragen.

Gefunden wurde ein Verfahren zur Aufreinigung von Ethoxyquin enthaltend p-Phenitidin umfassend mindestens einen Schritt der Destillation, bevorzugt umfassend keine weiteren Aufreinigungsschritte wie Adsorption, Wäsche, Kristallisation, besonders bevorzugt umfassend nur einen oder mehrere Destillationsschritte, ganz besonders bevorzugt umfassend nur einen oder zwei Destillationsschritte, wobei ein Ethoxyquin erhalten werden kann, das weniger als 100 ppm, bevorzugt weniger als 50 ppm, besonders bevorzugt weniger als 10 ppm und ganz besonders bevorzugt weniger als 5 ppm wie beispielsweise weniger als 1 ppm oder gar weniger als 0,5 ppm p-Phenitidin enthält sowie alle Werte dazwischen, beispielsweise weniger als 90, 80, 70, 60, 45, 40, 35, 30, 25, 20, 15, 9, 8, 7, 6, 4, 3, 2, 0,9, 0,8, 0,7, 0,6, 0,4, 0,3, 0,2 oder weniger als 0,1 enthält. Das Ethoxyquin erhältlich mittels des erfindungsgemäßen Verfahrens, enthält weniger als 100 ppm, bevorzugt weniger als 50 ppm, besonders bevorzugt weniger als 10 ppm und ganz besonders bevorzugt weniger als 5 ppm wie beispielsweise weniger als 1 ppm oder gar weniger als 0,5 ppm p-Phenitidin sowie alle Werte dazwischen, beispielsweise weniger als 90, 80, 70, 60, 45, 40, 35, 30, 25, 20, 15, 9, 8, 7, 6, 4, 3, 2, 0,9, 0,8, 0,7, 0,6, 0,4, 0,3, 0,2 oder weniger als 0,1. Das Ethoxyquin enthält weniger als 100 ppm, bevorzugt weniger als 50 ppm, besonders bevorzugt weniger als 10 ppm und ganz besonders bevorzugt weniger als 5 ppm wie beispielsweise weniger als 1 ppm oder gar weniger als 0,5 ppm p-Phenitidin sowie alle Werte dazwischen, beispielsweise weniger als 90, 80, 70, 60, 45, 40, 35, 30, 25, 20, 15, 9, 8, 7, 6, 4, 3, 2, 0,9, 0,8, 0,7, 0,6, 0,4, 0,3, 0,2 oder weniger als 0,1. Das Ethoxyquin, enthaltend weniger als 100 ppm, bevorzugt weniger als 50 ppm, besonders bevorzugt weniger als 10 ppm und ganz besonders bevorzugt weniger als 5 ppm wie beispielsweise weniger als 1 ppm oder gar weniger als 0,5 ppm p-Phenitidin sowie alle Werte dazwischen, beispielsweise weniger als 90, 80, 70, 60, 45, 40, 35, 30, 25, 20, 15, 9, 8, 7, 6, 4, 3, 2, 0,9, 0,8, 0,7, 0,6, 0,4, 0,3, 0,2 oder weniger als 0,1, ist nützlich als Zusatzstoff in Lebens- und Futtermitteln, bevorzugt in Futtermitteln, wobei Ethoxyquin beispielsweise Verwendung findet als Antioxidanz, besonders bevorzugt Verwendung als Antioxidanz in Futtermitteln und Futterzusatzstoffen.

Die Durchführung des Verfahrens kann dabei wie im Nachfolgenden erläutert in verschiedenen Maßstäben erfolgen je nach gewünschter Menge an Ethoxyquin. Das Grundprinzip und der grundsätzliche Aufbau der Apparatur ist dabei in beiden Fällen vergleichbar; Anpassungen ergeben sich vorallem aufgrund der Dimensionierung der Anlage. Ein eventuell nötiges Anpassen an die tatsächliche Dimensionierung und Ausgestaltung der Apparaturen ist im Rahmen der im nachfolgenden erläuterten Vorgaben für einen Fachmann ohne Weiteres möglich.

Die Destillation kann erfindungsgemäß diskontinuierlich oder kontinuierlich durchgeführt werden. Bevorzugt ist die kontinuierliche Durchführung. Insbesondere mittels kontinuierlicher Destillation können größere Mengen an erfindungsgemäßem Ethoxyquin bereitgestellt werden.

Im Rahmen der Bearbeitung eines Verfahrens zur Bereitstellung eines Produktes gemäß der vorliegenden Erfindung wurde erkannt, dass bei geschickter Durchführung der Destillation unter Verwendung einer sogenannten Trennwand-Kolonne schon eine einstufige Destillationsschritt ausreicht, um ein Ethoxyquin zu erhalten, das weniger als 100 ppm -Phenitidin enthält. Bei entsprechender Anpassung der Parameter und optional einem weiterem Destillationsschritt können so auch Ethoxyquin-Produkte erhalten werden, die extrem niedrige Anteile von p-Phenitidin bis nahezu kein p-Phenitidin mehr enthalten.

Eine solche geeignete Trennwandkolonne ist dem Fachmann bekannt, etwa aus US2471134, US4230533, EP122367A, EP 126288A, EP133510A, WO2010/031790, sowie aus Chem.Eng. Technol. 10, 1987, Seiten 92 bis 98, Chem.-Ing.-Tech. 61, 1989, Nr.1, Seiten 16-25, Gas Separation and Purification 4, 1990, Seiten 109 bis 114, Process Engineering 2, 1993, Seiten 33 und 34, Trans IChemE 72, 1994, Part A, Seiten 639 bis 644, Chemical Engineering 7, 1997, Seiten 72 bis 76.

Bei dieser Bauart ist im mittleren Bereich oberhalb und unterhalb der Zulaufstelle und der Seitenentnahme eine Trennwand angebracht, die den Zulaufteil 2, 4 gegenüber dem Entnahmeteil 3, 5 abdichtet und in diesem Kolonnenteil eine Quervermischung von Flüssigkeits- und Brüdenströmen unterbindet. Hierdurch verringert sich bei der Auftrennung von Vielstoffgemischen die Zahl der insgesamt benötigten Destillationskolonnen. Wie bei konventionellen Seitenabzugskolonnen können auch bei Trennwandkolonnen Zwischenverdampfer und Zwischenkondensatoren eingesetzt werden. Zwischenkondensatoren werden bevorzugt am oberen Ende der Trennwand oder im gemeinsamen Kolonnenbereich 1 oberhalb der Trennwand angebracht. Zwischenverdampfer werden bevorzugt am unteren Ende der Trennwand oder im gemeinsamen Kolonnenbereich 6 unterhalb der Trennwand vorgesehen.

Eine Trennwandkolonne kann bei gleichem Energieverbrauch auch durch die Anordnung von thermisch gekoppelten Destillationskolonnen ersetzt werden. Eine Beschreibung von thermisch gekoppelten Destillationskolonnen, die in verschiedener apparativer Gestaltung ausgeführt sein können, findet sich ebenfalls in den oben genannten Stellen in der Fachliteratur. Es ist auch möglich, die einzelnen Teilkolonnen komplett mit Verdampfern und Kondensatoren auszurüsten. Dies entspricht einer Trennwandkolonne mit einem Zwischenverdampfer und einem Zwischenkondensator. Ein besonderer Vorteil dieser speziellen Ausgestaltung ist, dass die einzelnen Kolonnen auch bei unterschiedlichen Drücken betrieben werden können. Dies ermöglicht es, zu hohe Temperaturspreizungen zu vermeiden und die Betriebstemperaturen besser an vorgegebene Heiz- und Kühlmedien anzupassen. Die Möglichkeiten für Energieverbundmaßnahmen werden verbessert.

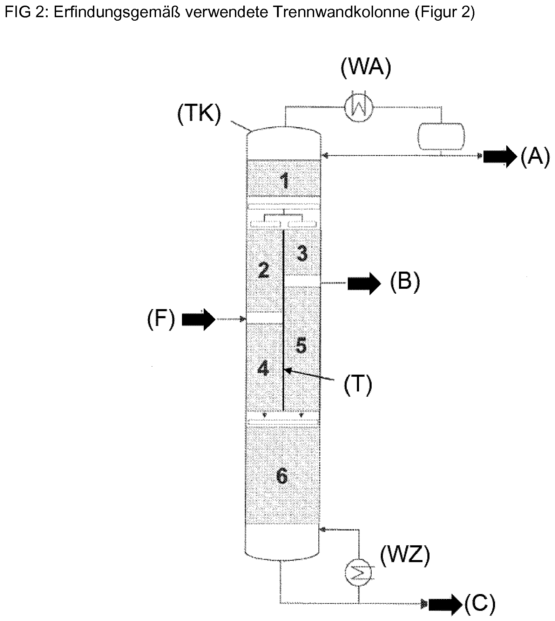

Eine weitere Bauform von erfindungsgemäß einsetzbaren Trennwandkolonnen sieht vor, die Trennwand durchgehend entweder bis zum oberen oder unteren Ende der Destillationskolonne auszuführen (Figur 2). Diese Bauform entspricht der Anordnung einer Hauptkolonne mit angeschlossener Seitenkolonne. Bei dieser Ausführungsform sind gegenüber konventionellen Kolonnenanordnungen keine Energie-, dafür aber Investitionskostenvorteile zu erwarten.

Trennwandkolonnen und thermisch gekoppelte Destillationskolonnen bieten gegenüber der Anordnung von konventionellen Destillationskolonnen sowohl hinsichtlich des Energiebedarfs als auch der Investitionskosten Vorteile. Für die Regelung von Trennwandkolonnen und thermisch gekoppelten Kolonnen werden verschiedene Regelungsstrategien beschrieben. Beschreibungen finden sich in: US 4,230,533, DE 35 22 234 C2, EP 780 147 A, Process Engineering 2 (1993), 33 - 34, und Ind. Eng. Chem. Res. 34 (1995), 2094 - 2103.

Eine typische, im erfindungsgemäßen Verfahren anzuwendende Trennwandkolonne (TK) (siehe Figur 2, aus WO2010/031790) weist jeweils eine Trennwand (T) in Kolonnenlängsrichtung unter Ausbildung eines oberen gemeinsamen Kolonnenbereichs (1), einen unteren gemeinsamen Kolonnenbereichs (6), eines Zulaufteils (2, 4) mit Verstärkungsteil (2) und Abtriebsteil (4), sowie eines Entnahmeteils (3, 5) mit Verstärkungsteil (5) und Abtriebsteil (3) auf, wobei die Zuführung des aufzutrennenden Gemischs (Feed) im mittleren Bereich des Zulaufteils (2, 4), die Abführung der Hochsiederfraktion über Sumpf (Sumpfabzug C), die Abführung der Leichtsiederfraktion über Kopf (Kopfabzug A) und die Abführung der Mittelsiederfraktion aus dem mittleren Bereich des Entnahmeteils (3, 5) (Seitenabzug B) erfolgt.

Die Trennwandkolonne/n des erfindungsgemäßen Verfahrens weist/weisen jeweils bevorzugt 30 bis 100, insbesondere 50 bis 90, theoretische Trennstufen auf.

Eine typische Trennwandkolonne im Rahmen der vorliegenden Erfindung weist bevorzugt jeweils eine Trennwand (T) in Kolonnenlängsrichtung unter Ausbildung eines oberen (C) und unteren (D) gemeinsamen Kolonnenbereichs, eines Zulaufteils (A) und Entnahmeteils (B). Die Zuführung des aufzutrennenden Gemischs erfolgt im mittleren Teil des Zulaufbereichs, die Abführung des Produktes EQ im mittleren Bereich des Entnahmeteils.

Insbesondere weist im erfindungsgemäßen Verfahren der obere gemeinsame Kolonnenbereich (1) der Trennwandkolonne/n (TK) 5 bis 50 %, bevorzugt 20 bis 35 %, der Verstärkungsteil (2) des Zulaufteils (2, 4) der Kolonne 5 bis 50 %, bevorzugt 10 bis 20 %, der Abtriebsteil (4) des Zulaufteils der Kolonne 5 bis 50 %, bevorzugt 20 bis 35 %, der Verstärkungsteil (3) des Entnahmeteils (3, 5) der Kolonne 5 bis 50 %, bevorzugt 7 bis 20 %, der Abtriebsteil (5) des Entnahmeteils der Kolonne 5 bis 50 %, bevorzugt 20 bis 35 %, und der gemeinsame untere Bereich (6) der Kolonne 5 bis 50 %, bevorzugt 20 bis 35 %, der Gesamtzahl der theoretischen Trennstufen (nth) der Kolonne auf. Die Gesamtsumme ergibt sich dabei zu 100 (einhundert) Prozent.

Insbesondere beträgt in der bzw. den Trennwandkolonne/n (TK) jeweils die Summe der Zahl der theoretischen Trennstufen der Teilbereiche (2) und (4) im Zulaufteil 80 bis 110 %, bevorzugt 90 bis 100 %, der Summe der Zahl der Trennstufen der Teilbereiche (3) und (5) im Entnahmeteil. Die Gesamtsumme ergibt sich dabei zu 100 (einhundert) Prozent.

Der Trennwandteil weist bevorzugt 40-80%, bevorzugt 50-70%, der obere gemeinsame Kolonnenteil 5-50% bevorzugt etwa 15-20%, der untere gemeinsame Kolonnenteil 5-50% bevorzugt etwa 15-20% der Gesamtzahl der theoretischen Trennstufen der Kolonne auf. Die Gesamtsumme ergibt sich dabei zu 100 (einhundert) Prozent.

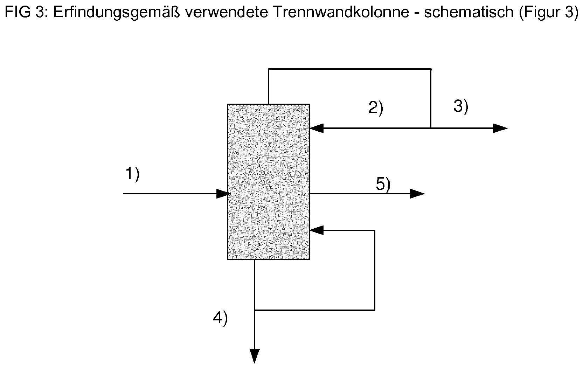

Die Trennwandkolonne bestehend aus einer Kolonne (wie oben beschrieben) mit einem Wärmetauscher als Verdampfer und einem Wärmetauscher als Kondensator (siehe Figur 3). Die Kolonnenflüsse sind (siehe Figur 3): 1) Zulaufstrom zu der Kolonne, 3) Destillat definiert als eine definierte Teilmenge des kondensierten Brüdenstroms, der 5) Seitenabzug, und 4) der Sumpfabzug definiert als einer definierten Teilmenge des Sumpfstroms. Der Rückfluss 2) der Kolonne definiert als eine definierte Teilmenge des kondensierten Brüdenstroms, welche auf den oberen gemeinsamen Teil der Kolonne (C) zurückgeführt wird. Der Sumpfstrom wurde über einen Verdampfer bei 190-220°C verdampft und dem unteren gemeinsamen Teil der Kolonne (D) zugeführt. Verdampfer als solches, Vor- und Nachteile verschiedener Verdampfer-Typen sowie deren Anwendung sind dem Fachmann hinlänglich bekannt, etwa aus dem Lexikon Römpp (Thieme-Verlag), Stichwort "Dünnschichtverdampfer" (etwa die Aktualisierung von August 2004); dort werden beispielsweise Fallfilmverdampfer/Fallstromverdampfer, Rotationsdünnschichtverdampfer, Zentrifugaldünnschichtverdampfer kurz dargestellt. Weitere entsprechende grundlegende Fachliteratur ist auch Vauck und Müller "Grundoperationen chemischer Verfahrenstechnik, 11.Auflage, 2000, Deutscher Verlag für Grundstoffindustrie; Sattler "Thermische Trennverfahren", 2.Auflage, 2001, Wiley-VCH; Gnielinski, Mersmann, Thurner, "Verdampfung, Kristallisation, Trocknung", Vieweg-Verlag 1993.

Falls besonders hohe Anforderungen an die Reinheiten der Ethoxyquin-Produkte gestellt werden, ist es günstig und im Rahmen dieser Erfindung ganz besonders bevorzugt, die Trennwand mit einer thermischen Isolierung auszustatten. Eine Beschreibung der verschiedenen Möglichkeiten der thermischen Isolierung der Trennwand findet sich z. B. in EP 640 367 A. Eine doppelwandige Ausführung mit einem zwischenliegenden engen Gasraum ist besonders günstig und daher insbesondere bevorzugt.

Bevorzugt ist der durch die Trennwand (T) unterteilte Teilbereich der Trennwandkolonne/n (TK) bestehend aus den Teilbereichen 2, 3, 4 und 5 oder Teilen davon mit geordneten Packungen oder Füllkörpern bestückt und die Trennwand in diesen Teilbereichen bevorzugt wärmeisolierend ausgeführt.

Bevorzugt ist weiterhin, dass die Trennwandkolonne/n (TK) am oberen und unteren Ende der Trennwand (T) Probenahmemöglichkeiten aufweist/aufweisen und aus der/den Kolonne kontinuierlich oder in zeitlichen Abständen flüssig oder gasförmig Proben entnommen und hinsichtlich ihrer Zusammensetzung untersucht werden.

Die Einhaltung der Spezifikation für die Hochsieder in einer Mittelsiederfraktion kann beispielsweise über das Aufteilungsverhältnis der Flüssigkeit am oberen Ende der Trennwand geregelt. Dabei wird das Aufteilungsverhältnis der Flüssigkeit am oberen Ende der Trennwand/Trennwände (T) so eingestellt, dass die Konzentration der Schlüsselkomponenten für die Hochsiederfraktion in der Flüssigkeit am oberen Ende der Trennwand beispielsweise 5 bis 75 %, üblicherweise 10 bis 40 %, des Wertes ausmacht, der im Seitenabzugsprodukt erzielt werden soll, und die Flüssigkeitsaufteilung dahingehend eingestellt wird, dass bei höheren Gehalten an Schlüsselkomponenten der Hochsiederfraktion mehr und bei niedrigeren Gehalten an Schlüsselkomponenten der Hochsiederfraktion weniger Flüssigkeit auf den Zulaufteil geleitet wird.

Entsprechend kann die Spezifikation für die Leichtsieder in der Mittelsiederfraktion bevorzugt über die die Heizleistung geregelt werden. Hierbei wird die Heizleistung im Verdampfer der jeweiligen Trennwandkolonne so eingestellt, dass die Konzentration an Schlüsselkomponenten der Leichtsiederfraktion in der Flüssigkeit am unteren Ende der Trennwand/Trennwände (T) beispielsweise 10 bis 99 %, wie etwa 25 bis 97,5 %, des Wertes ausmacht, der im Seitenabzugsprodukt erzielt werden soll, und die Heizleistung kann dahingehend eingestellt werden, dass bei höherem Gehalt an Schlüsselkomponenten der Leichtsiederfraktion die Heizleistung erhöht und bei niedrigerem Gehalt an Schlüsselkomponenten der Leichtsiederfraktion die Heizleistung verringert wird. Ein solches Vorgehen ist dem Fachmann prinzipiell bekannt. Eine Anpassung an die jeweils verwendete Kolonne bei der Reinigung von Ethoxyquin kann der Fachmann somit auf Basis der in der vorliegenden Offenbarung gemachten Hinweise selbständig vornehmen.

Eine weitere erfindungsgemäße Variation des Verfahrens zur destillativen Aufarbeitung von Ethoxyquin besteht darin, dass anstelle einer der genannten Trennwandkolonne/n - die bei einem Neubau hinsichtlich der Investitionskosten zu bevorzugen ist/sind - eine Zusammenschaltung von zwei (konventionellen) Destillationskolonnen in Form einer thermischen Kopplung verwendet wird (thermisch gekoppelte Kolonnen, die hinsichtlich des Energiebedarfs einer Trennwandkolonne entsprechen). Dies ist vor allem dann günstig, wenn die Kolonnen schon vorhanden sind und/oder die Kolonnen bei verschiedenen Drücken betrieben werden sollen. Je nach der Trennstufenzahl der vorhandenen Kolonnen können die geeignetsten Formen der Zusammenschaltung gewählt werden.

Bevorzugt sind beide thermisch gekoppelten Destillationskolonnen jeweils mit einem eigenen Verdampfer und Kondensator ausgestattet.

Weiterhin werden bevorzugt die beiden thermisch gekoppelten Kolonnen bei verschiedenen Drücken betrieben und in den Verbindungsströmen zwischen den beiden Kolonnen nur Flüssigkeiten gefördert. Es ist also möglich, Schaltungsformen zu wählen, die es erlauben, dass nur flüssige Verbindungsströme zwischen den einzelnen Destillationskolonnen auftreten.
Diese speziellen Verschaltungen bieten den Vorteil, dass die beiden Destillationskolonnen unter verschiedenen Drücken betrieben werden können mit dem Vorteil, dass sie sich besser an die Temperaturniveaus vorhandener Heiz- und Kühlenergien anpassen lassen.
Bevorzugt wird der Sumpfstrom der ersten Kolonne der beiden thermisch gekoppelten Kolonnen in einem zusätzlichen Verdampfer teilweise oder vollständig verdampft und anschließend der zweiten Kolonne zweiphasig oder in Form eines gasförmigen und eines flüssigen Stromes zugeführt. Im erfindungsgemäßen Verfahren wird bevorzugt der Zulaufstrom (Feed) zur Kolonne/zu den Kolonnen teilweise oder vollständig vorverdampft und der/den Kolonne/n zweiphasig oder in Form eines gasförmigen und eines flüssigen Stromes zugeführt.

Trennwandkolonnen und thermisch gekoppelte Kolonnen können als Packungskolonnen mit Füllkörpern oder geordneten Packungen oder als Bodenkolonnen ausgeführt werden. Besonders bevorzugt im erfindungsgemäßen Verfahren ist die Verwendung einer Trennwand-kolonne.

Folgende Parameter zur Auslegung der Trennwandkolonne wurden erfindungsgemäß bevorzugt verwendet, wobei diese Parameter jeweils einzeln ausgewählt und die jeweiligen Auswahlen prinzipiell miteinander kombiniert werden können, wobei etwaige Abhängigkeiten entweder in dieser Offenbarung dargestellt werden oder sich inhärent ergeben:
- Trennstufenkolonne mit einer Zahl an Trennstufen von bevorzugt 10 bis100, besonders bevorzugt 20 bis 100, ganz besonders bevorzugt 30 bis 70;
- Betreiben der Destillationskolonne bei 1 bis 100 mbar, bevorzugt 1 bis 50, besonders bevorzugt 1 bis 20, ganz besonders bevorzugt 1 bis10, insbesondere 1 bis 5;
- Die Sumpftemperatur der Kolonne wird auf Werte von 80 bis 200°C eingestellt, bevorzugt von etwa 100 bis 200, besonders bevorzugt von 150 bis 200, ganz besonders bevorzugt von 190 bis 195°C;
- Die Sumpfflüssigkeitsmenge (Volumen) beträgt von 1 bis 30% des Gesamtvolumens an zu trennender Stoffmenge in der Kolonne), bevorzugt von 1 bis 20%, besonders bevorzugt von 1 bis 15 wie beispielweise von 1 bis 10;.
- Das Verhältnis von Sumpfabzugsmenge zu Zulaufmenge beträgt von 0,01 bis 0,3, bevorzugt von 0,05 bis 0,1;
- Einsatz von Packungskolonnen, insbesondere geordneten Packungen; Bei der erfindungsgemäßen Reindestillation von Ethoxyquin, die bevorzugt im Vakuum betrieben wird, empfiehlt es sich, Packungskolonnen einzusetzen. Dabei sind geordnete Packungen mit einer spezifischen Oberfläche von 100 bis 1000 m²/m³, bevorzugt etwa 250 bis 900 m²/m³, besonders bevorzugt etwa 400 bis 800 m²/m³, ganz besonders bevorzugt etwa 500 bis 750 m²/m³, geeignet. Solche Packungen sind bevorzugt aus Blech.
- Das Verhältnis von Rücklaufmenge (Figur 3, Nummer 2) zu Zulaufmenge (Figur 3, Nummer 1) weist einen Faktor von 0,5:1 bis 5:1, bevorzugt 1:1 bis 2:1 und besonders bevorzugt 1,3:1 auf;

Der Seitenabzug enthält bei Durchführung des erfindungsgemäßen Verfahrens Ethoxyquin mit einer Reinheit von bevorzugt mehr als 90 bis 99% oder mehr, besonders bevorzugt mehr als 95 bis 99% oder mehr, wie beispielsweise 99,5, 99,9, 99,99, bis nahezu 100 Prozent.
Der Phenetidin Gehalt im Seitenabzug beträgt bevorzugt weniger als 100 ppm, bevorzugt weniger als 50 ppm, besonders bevorzugt weniger als 10 ppm und ganz besonders bevorzugt weniger als 5 ppm wie beispielsweise weniger als 1 ppm oder gar weniger als 0,5 ppm p-Phenitidin enthält sowie alle Werte dazwischen, beispielsweise weniger als 90, 80, 70, 60, 45, 40, 35, 30, 25, 20, 15, 9, 8, 7, 6, 4, 3, 2, 0,9, 0,8, 0,7, 0,6, 0,4, 0,3, 0,2 oder weniger als 0,1.

Das nach dem erfindungsgemäßen Vorgehen erhaltene, erfindungsgemäße Ethoxyquin kann weitere Nebenkomponenten außer p-Phenitidin enthalten. Diese stammen üblicherweise aus der Synthese des Ethoxyquins und deren Vorprodukten, und/oder aus Zersetzungsprozessen dieser Substanzen. Das vorliegende Verfahren kann jedoch auch diese Nebenkomponenten weitgehend reduzieren gemäß der zuvor genannten Reinheiten von Ethoxyquin.

Bevorzugt wird das Verfahren zur Reinigung von Ethoxyquin erfindungsgemäß so durchgeführt, dass die Verweilzeit des Schwersieders im Sumpf weniger als 10 Stunden, besonders bevorzugt weniger als 8 Stunden, ganz besonders bevorzugt weniger als 5 Stunden, sowie alle Werte dazwischen und bis zu 1 Stunde, beträgt, beispielsweise 9,5, 9, 8,5, 8, 7,5, 7, 6,5, 6, 5,5, 4,5, 4, 3,5, 3, 2,5, 2, 1,5 oder 1.

Bei einer erhöhten Verweilzeit wurde ein erhöhter Phenetidin Gehalt im Sumpf und im Seitenabzug festgestellt, so dass ein Ethoxyquin erhalten wurde, das deutlich mehr als die erfindungsgemäßen Mengen von maximal 100 ppm p-Phenitidin enthielt.

Besonders bevorzugte Parameter zur Steuerung des erfindungsgemäßen Verfahrens und zum Erhalt des erfindungsgemäßen Ethoxyquins sind die folgenden:
1) Sumpfflüssigkeitstand
2) Sumpfabzugsmenge,
3) Verwendetes Vakuum,
4) verwendete Stufenzahl

Diese vier wichtigsten Parameter können einzeln, oder mehrere oder alle gleichzeitig angepasst werden anhand der zuvor offenbarten erfindungsgemäßen Bereichen für diese Parameter sowie deren jeweilige Bevorzugungen. Bevorzugt ist, die jeweils bevorzugten, besonders bevorzugt die jeweils besonders bevorzugten, ganz besonders bevorzugt die jeweils ganz besonders bevorzugten und insbesondere bevorzugt die jeweils insbesondere bevorzugten Bereiche dieser vier Parameter zu kombinieren.

Alle weiteren Parameter können ebenfalls entsprechend zugewählt werden, wobei bevorzugt jeweils die bevorzugten Bereiche, besonders bevorzugt die jeweils besonders bevorzugten, ganz besonders bevorzugt die jeweils ganz besonders bevorzugten und insbesondere bevorzugt die jeweils insbesondere bevorzugten Bereiche dieser weiteren Parameter mit den vier zuvor als besonders bevorzugt genannten Parametern (Sumpfflüssigkeitstand, Sumpfabzugsmenge, Verwendetes Vakuum, verwendete Stufenzahl) und deren jeweiligen bevorzugten, besonders bevorzugten, ganz besonders bevorzugten und insbesondere bevorzugten Kombinationen zu kombinieren.

Selbstverständlich kann aber auch jeweils jeder der Parameter insoweit unabhängig von den anderen Parametern so eingestellt werden, dass der eingestellte Wert für einen einzustellenden Parameter in den allgemeinen, den bevorzugten, den besonders bevorzugten oder den insbesondere bevorzugten Bereich fällt, wobei die sich ergebenden Abhängigkeiten der Parameter wie zuvor geschildert oder sich dann offensichtlich ergebend die freie Wahl für den jeweils einzeln betrachteten Parameter einschränken können. Diese Einschränkungen ergeben sich aber gegebenenfalls zwangsläufig und sind damit ein inhärentes Merkmal der Erfindung und bedürfen somit auch keiner besonderen Erklärung.

Weiterhin ist es möglich - und im Rahmen dieser Erfindung bevorzugt - wenigstens beim Anfahren der Kolonne (bzw. die entsprechende Apparatur zur Destillation), besonders bevorzugt zusätzlich auch während des Betriebs, und ganz besonders bevorzugt nur zum Anfahren der Kolonne, den Zulauf zu erwärmen. Dieses Erwärmen findet statt auf Temperaturen von wenigstens 50 °C auf Temperaturen von bis zu 200 °C, bevorzugt von 80 bis 190, besonders bevorzugt von 100 bis 180 und ganz besonders bevorzugt von 120 bis 170 und insbesondere auf 140 bis 160 °C.

In einer alternativen Ausführungsform kann anstelle des Erwärmens des Zulaufs auch die Kolonne selbst durch eine geeignete Flüssigkeit erwärmt werden. Dazu wird diese Flüssigkeit, etwa ein Lösemittel erwärmt in die Kolonne gefahren und somit die Kolonne im Innern wie auch die Zu- und Abläufe und Abzüge erhitzt auf die gewünschte Temperatur, bevor dann das zu reinigende Ethoxyquin in die Kolonne (bzw. die entsprechende Apparatur zur Destillation) gefahren wird.

Neben thermisch isolierten Kolonnen etwa durch doppel- oder mehrwandig ausgeführte Kolonnenwände oder Teilen davon sowie gegebenenfalls zusätzlich ganz oder teilweise isolierten Rohren von Zu- und Abläufen und Abzügen ist es ebenfalls denkbar, eine sogenannte Begleitheizung zu verwenden.

Begleitheizung bedeutet, dass alle oder wenigstens die zentralen Teile von Kolonne sowie Rohre der Zu- und Abläufe und der Abzüge beheizt werden. Eine Beheizung kann von außen erfolgen (durch Heizmäntel jeglicher Art oder Direktbeheizung durch offene Flammen) oder auch durch elektrische (wie etwa induktive) Heizung der Metallteile etwa durch integrierte oder aufgebrachte Heizschlangen oder elektrische Leiter oder durch induktive Beheizung.

Weitere bevorzugte Ausführungsformen neben den zuvor beschriebenen sind die folgenden Ausführungsformen 1 bis 18 und die jeweils genannten Möglichkeiten der Kombination und Rückbezüge:
Ausführungsform 1: Verfahren zur Aufreinigung von Ethoxyquin enthaltend p-Phenitidin umfassend mindestens einen Schritt der kontinuierlichen oder der diskontinuierlichen Destillation unter Verwendung wenigstens einer Trennwandkolonne oder wenigstens zwei thermisch gekoppelten Kolonnen, wobei ein Ethoxyquin erhalten wird, das weniger als 100 ppm, bevorzugt weniger als 50 ppm, besonders bevorzugt weniger als 10 ppm und ganz besonders bevorzugt weniger als 5 ppm wie beispielsweise weniger als 1 ppm oder gar weniger als 0,5 ppm p-Phenitidin enthält sowie alle Werte dazwischen, beispielsweise weniger als 90, 80, 70, 60, 45, 40, 35, 30, 25, 20, 15, 9, 8, 7, 6, 4, 3, 2, 0,9, 0,8, 0,7, 0,6, 0,4, 0,3, 0,2 oder weniger als 0,1 enthält.
Ausführungsform 2 Verfahren nach Ausführungsform 1, umfassend keine weiteren Aufreinigungsschritte wie Adsorption, Wäsche, Kristallisation.
Ausführungsform 3 Verfahren nach Ausführungsform 1 oder 2, wobei die wenigstens eine Kolonne eine Trennwandkolonne ist oder wenigstens eine der wenigstens zwei gekoppelten Kolonnen eine Trennwandkolonne ist.
Ausführungsform 4 Verfahren nach Ausführungsform 3, wobei die Trennwandkolonne eine Trennwand (T) in Kolonnenlängsrichtung unter Ausbildung eines oberen (C) und unteren (D) gemeinsamen Kolonnenbereichs, eines Zulaufteils (A) und Entnahmeteils (B) aufweist, und die Zuführung des aufzutrennenden Gemischs im mittleren Teil des Zulaufbereichs sowie die Abführung des Produktes Ethoxyquin im mittleren Bereich des Entnahmeteils erfolgt.
Ausführungsform 5 Verfahren nach Ausführungsform 3 oder 4, wobei die Trennwand der Trennwandkolonne durchgehend entweder bis zum oberen oder bis zum unteren Ende der Destillationskolonne oder bis zum oberen und zum unteren Ende ausgeführt ist.
Ausführungsform 6 Verfahren nach einer der vorigen Ausführungsformen, wobei nur eine Kolonne und diese in Form einer Trennwandkolonne eingesetzt wird.
Ausführungsform 7 Verfahren nach einer der vorigen Ausführungsformen, wobei die Trennwandkolonne (TK) eine Trennwand (T) in Kolonnenlängsrichtung unter Ausbildung eines oberen gemeinsamen Kolonnenbereichs (1), einen unteren gemeinsamen Kolonnenbereichs (6), eines Zulaufteils (2, 4) mit Verstärkungsteil (2) und Abtriebsteil (4), sowie eines Entnahmeteils (3, 5) mit Verstärkungsteil (5) und Abtriebsteil (3) aufweist, wobei die Zuführung des aufzutrennenden Gemischs (Feed) im mittleren Bereich des Zulaufteils (2, 4), die Abführung der Hochsiederfraktion über Sumpf (Sumpfabzug C), die Abführung der Leichtsiederfraktion über Kopf (Kopfabzug A) und die Abführung der Mittelsiederfraktion aus dem mittleren Bereich des Entnahmeteils (3, 5) (Seitenabzug B) erfolgt.
Ausführungsform 8 Verfahren nach einer der vorigen Ausführungsformen, wobei die Trennwandkolonne/n 30 bis 100, insbesondere 50 bis 90 theoretische Trennstufen aufweisen.
Ausführungsform 9 Verfahren nach einer der vorigen Ausführungsformen, wobei der obere gemeinsame Kolonnenbereich (1) der Trennwandkolonne/n (TK) 5 bis 50 %, bevorzugt 20 bis 35 %, der Verstärkungsteil (2) des Zulaufteils (2, 4) der Kolonne 5 bis 50 %, bevorzugt 10 bis 20 %, der Abtriebsteil (4) des Zulaufteils der Kolonne 5 bis 50 %, bevorzugt 20 bis 35 %, der Verstärkungsteil (3) des Entnahmeteils (3, 5) der Kolonne 5 bis 50 %, bevorzugt 7 bis 20 %, der Abtriebsteil (5) des Entnahmeteils der Kolonne 5 bis 50 %, bevorzugt 20 bis 35 %, und der gemeinsame untere Bereich (6) der Kolonne 5 bis 50 %, bevorzugt 20 bis 35 %, der Gesamtzahl der theoretischen Trennstufen (nth) der Kolonne aufweist.
Ausführungsform 10 Verfahren nach einer der vorigen Ausführungsformen, wobei in der bzw. den Trennwandkolonne/n (TK) jeweils die Summe der Zahl der theoretischen Trennstufen der Teilbereiche (2) und (4) im Zulaufteil 80 bis 110 %, bevorzugt 90 bis 100 %, der Summe der Zahl der Trennstufen der Teilbereiche (3) und (5) im Entnahmeteil beträgt.
Ausführungsform 11: Verfahren nach einer der vorigen Ausführungsformen, wobei der Trennwandteil 40-80%, bevorzugt 50-70%, der obere gemeinsame Kolonnenteil 5-50% bevorzugt etwa 15-20%, der untere gemeinsame Kolonnenteil 5-50% bevorzugt etwa 15-20% der Gesamtzahl der theoretischen Trennstufen der Trennwandkolonne aufweist.
Ausführungsform 12 Verfahren nach einer der vorigen Ausführungsformen, wobei die Kolonne(n) mindestens einen Wärmetauscher als Verdampfer und mindestens einen Wärmetauscher als Kondensator je verwendeter Kolonne aufweist/aufweisen.
Ausführungsform 13 Verfahren nach einer der vorigen Ausführungsformen, wobei die wenigstens eine Kolonne jeweils doppel- oder mehrwandig, bevorzugt doppelwandig, mit mindestens einem zwischen den mindestens zwei Wänden liegenden Gasraum, ausgeführt ist.
Ausführungsform 14 Verfahren nach einer der vorigen Ausführungsformen, wobei der obere gemeinsame Kolonnenbereich (1) der Trennwandkolonne/n (TK) 5 bis 50 %, bevorzugt 20 bis 35 %, der Verstärkungsteil (2) des Zulaufteils (2, 4) der Kolonne 5 bis 50 %, bevorzugt 10 bis 20 %, der Abtriebsteil (4) des Zulaufteils der Kolonne 5 bis 50 %, bevorzugt 20 bis 35 %, der Verstärkungsteil (3) des Entnahmeteils (3, 5) der Kolonne 5 bis 50 %, bevorzugt 7 bis 20 %, der Abtriebsteil (5) des Entnahmeteils der Kolonne 5 bis 50 %, bevorzugt 20 bis 35 %, und der gemeinsame untere Bereich (6) der Kolonne 5 bis 50 %, bevorzugt 20 bis 35 %, der Gesamtzahl der theoretischen Trennstufen (nth) der Kolonne aufweist, und wobei der durch die Trennwand (T) unterteilte Teilbereich der Trennwandkolonne(n) (TK) bestehend aus den Teilbereichen 2, 3, 4 und 5 oder Teilen davon mit geordneten Packungen oder Füllkörpern bestückt ist, und die Kolonne(n) wenigstens teilweise mit Füllkörpern oder geordneten Packungen, bevorzugt geordneten Packungen, besonders bevorzugt solchen aus Blech, gefüllt ist
Ausführungsform 15 Verfahren nach einer der vorigen Ausführungsformen, wobei
   A) die Trennstufenkolonne eine Zahl an Trennstufen von bevorzugt 10 bis100, besonders bevorzugt 20 bis 100, ganz besonders bevorzugt 30 bis 70, aufweist;
   B) die Destillationskolonne bei 1 bis 100 mbar, bevorzugt 1 bis 50, besonders bevorzugt 1 bis 20, ganz besonders bevorzugt 1 bis10, insbesondere 1 bis 5 betrieben wird;
   C) die Sumpftemperatur der Kolonne auf Werte von 80 bis 200°C, bevorzugt von etwa 100 bis 200, besonders bevorzugt von 150 bis 200, ganz besonders bevorzugt von 190 bis 195°C eingestellt wird;
   D) die Sumpfflüssigkeitsmenge (das Volumen) von 1 bis 30% des Gesamtvolumens an zu trennender Stoffmenge in der Kolonne), bevorzugt von 1 bis 20%, besonders bevorzugt von 1 bis 15 wie beispielweise von 1 bis 10 beträgt;
   E) das Verhältnis von Sumpfabzugsmenge zu Zulaufmenge von 0,01 bis 0,3, bevorzugt von 0,05 bis 0,1 beträgt;
   F) Packungskolonnen, insbesondere geordnete Packungen, und insbesondere Blechpackungen, verwendet werden mit einer spezifischen Oberfläche von 100 bis 1000 m²/m³, bevorzugt etwa 250 bis 900 m²/m³, besonders bevorzugt etwa 400 bis 800 m²/m³, ganz besonders bevorzugt etwa 500 bis 750 m²/m³, verwendet werden; und
   G) das Verhältnis von Rücklaufmenge zu Zulaufmenge einen Faktor von 0,5:1 bis 5:1, bevorzugt 1:1 bis 2:1 und besonders bevorzugt 1,3:1 aufweist.
Ausführungsform 16 Verfahren nach Ausführungsform 15, wobei die Verweilzeit des Schwersieders im Sumpf weniger als 10 Stunden, besonders bevorzugt weniger als 8 Stunden, ganz besonders bevorzugt weniger als 5 Stunden, sowie alle Werte dazwischen und bis zu 1 Stunde, beträgt, beispielsweise 9,5, 9, 8,5, 8, 7,5, 7, 6,5, 6, 5,5, 4,5, 4, 3,5, 3, 2,5, 2, 1,5 oder 1.
Ausführungsform 17 Verfahren nach einer der vorigen Ausführungsformen, wobei die Kolonne(n) begleitbeheizt werden.
Ausführungsform 18 Verfahren nach einer der vorigen Ausführungsformen, wobei wenigstens beim Anfahren der Zulauf vorerwärmt wird auf eine Temperatur von wenigstens 50 °C bis zu 200 °C, bevorzugt von 80 bis 190, besonders bevorzugt von 100 bis 180 und ganz besonders bevorzugt von 120 bis 170 und insbesondere auf 140 bis 160 °C.

Die Laboranalytik des p-Phenetidin und EQ erfolgt via Gaschromatografie. Die entsprechenden Verweilzeiten und die Methodik sind dem Fachmann bekannt, und kann gegebenenfalls auch sehr einfach ermittelt und validiert werden. Die prinzipielle Vorgehensweise dazu einschliesslich der Validierung ist fachbekannt. Somit sind jegliche Methoden zur Gaschromotagrafie geeignet, sofern sie eine entsprechende Genauigkeit für p-Phenitidin in Ethoxyquin liefern, was durch eine Validierung mit geeichten Probenmustern enthalten Ethoxyquin und genormte Mengen p-Phenitidin ohne weiteres ermittelt werden kann.

### Beispiele

Erfindungsgemäße Versuche - Tabelle 1: Variation Destillationsparameter Labordestillation an Trennwandkolonne; Ethoxyquin ("Roh"-Destillat; enthält 0,18 g (= 1800 ppm) p-Phenitidin)

**(Tabelle 1)**

| Versuch | Zulauf | Rücklauf | Druc k Kopf | Temp. Sumpf | Tem p.Ko pf | Temp. Hauptkondensator | Kopfabzug | Sump f-abzug | Seitenabzug | Phenetidin im Produkt des Seitenabzugs |
|---|---|---|---|---|---|---|---|---|---|---|
| | g/h | g/h | [mbar ] | °C | C | C | g/h | g/h | g/h | [ppm] |
| 1 | 302 | 466 | 12 | 168 | 160 | 60 | 65 | 104 | 215 | 5 |
| 2 | 301 | 442 | 12 | 171 | 159 | 60 | 30 | 22 | 236 | 5 |
| 3 | 284 | 403 | 12 | 172 | 158 | 60 | 25 | 22 | 234 | 5 |
| 4 | 274 | 400 | 17 | 179 | 164 | 60 | 25 | 20 | 228 | 6 |
| 5 | 284 | 400 | 22 | 188 | 169 | 60 | 26 | 31 | 225 | 5 |
| 6 | 285 | 399 | 30 | 196 | 175 | 90 | 26 | 30 | 226 | 8 |
| 7 | 284 | 421 | 35 | 201 | 179 | 90 | 30 | 26 | 226 | 8 |
| 8 | 247 | 406 | 35 | 200 | 179 | 90 | 28 | 37 | 182 | 6 |
| 10* | 250 | 399 | 35 | 198 | 178 | 90 | 26 | 40 | 183 | 3 |

| | | | | | | | | | | |
|---|---|---|---|---|---|---|---|---|---|---|
| * Versuch 10 mit vorgeschaltetem Dünnschichtverdampfer | | | | | | | | | | |

Aus den Versuchen 1 bis 10 ist weiterhin abzuleiten, dass es erstrebenswert ist, eine niedrige thermische Belastung (i.e. niedrige Temperatur) bei der Destillation einzustellen, um Zersetzungsreaktionen von Schwersiedern, meist im Sumpf, zu vermeiden. Weiterhin soll bevorzugt die Verweilzeit der Komponenten im Sumpf gering gehalten werden.

Nicht erfindungsgemäßes Beispiel:
Abreicherung von p-Phenitidin aus Ethoxyquin mittels saurer Extraktion

Prinzipielles Vorgehen: Die organische Phase enthaltend "roh"-destilliertes Ethoxyquin (enthält 0,18 g - entsprechend 1800 ppm - p-Phenitidin) wurde mehrmals mittels wässrig saurer Lösung gewaschen. Dann erfolgte die Phasentrennung. Die Phasen wurden beprobt auf Ethoxyquin- und p-Phenitidin-Gehalte.

Konkretes Vorgehen der durchgeführten Versuche:
500mL Ethoxyqin, 250 mL Wasser, 23g wässrige Phosphorsäure (Gehalt 85%) wurden über Nacht gerührt. Zugabe von 23g wässriger Phosphorsäure (Gehalt 85%) Die entstandenen Phasen wurden getrennt (Oberphase organisch, Unterphase wässrig)
- Unterphase: mit etwa 200 ml Natronlauge (Gehalt 25 %) auf pH12 gestellt.
- Oberphase (organisch): Zugabe von 250 ml Wasser und 23 g wässriger Phosphorsäure (Gehalt 85%); Rühren über Nacht. Phasentrennung; erneute Unterphase (wässrig) wurde abgetrennt; Zugabe zur erneuten Unterphase von etwa 200mL Natronlauge (Gehalt 25%) auf pH 12.

Nach Extraktion wurde sowohl das zur sauren Extraktion eingesetzte Ethoxyquin als auch der Extrakt auf die Gehalte an Ethoxyquin und Phenitidin untersucht. Es wurde keine signifikante Ab- bzw. Anreicherung gefunden, das heisst der Wert für p-Phenitin lag im gleichen Größenbereich wie vor der Behandlung.

Erklärung zur Figur 2 : (A) = Kopfabzug; (B) = Seitenabzug; (C) = Sumpfabzug; (F) = Feed (Zuführung); (T) = Trennwand; (WZ) = Wärmezufuhr; (WA) = Wärmeabfuhr; (TK) = Trennwandkolonne; oberer gemeinsamer Kolonnenbereich (1); unterer gemeinsamer Kolonnenbereich (6); Zulaufteil (2, 4) mit Verstärkungsteil (2) und Abtriebsteil (4); Entnahmeteil (3, 5) mit Verstärkungsteil (5) und Abtriebsteil (3); Zuführung des aufzutrennenden Gemischs (Feed) (F) im mittleren Bereich des Zulaufteils (2, 4);

Trennwandkolonne Schematisch (Figur 3); Erklärung:
Feed (Zuführung) 1); Rückfluss (Teilmenge des Brüdenstroms) 2); Kopfabzug 3); Sumpfabzug mit teilweiser Rückführung 4) in den unteren gemeinsamen Teil der Kolonne; Seitenabzug 5); Zulaufteil (A); Entnahmeteil (B); oberer gemeinsamer Teil der Kolonne (C); unterer gemeinsamer Teil der Kolonne (D); Trennwand (T)

## Patentansprüche

1. Verfahren zur Aufreinigung von Ethoxyquin enthaltend p-Phenitidin, umfassend mindestens einen Schritt der kontinuierlichen Destillation unter Verwendung wenigstens einer Trennwandkolonne oder wenigstens zweier thermisch gekoppelten Kolonnen,
wobei wenigstens eine der wenigstens zwei gekoppelten Kolonnen eine Trennwandkolonne ist, wobei ein Ethoxyquin erhalten wird, das weniger als 100 ppm p-Phenitidin enthält, wobei
A) die wenigstens eine Kolonne eine Zahl an Trennstufen von 10 bis 100 aufweist;
B) die wenigstens eine Kolonne bei 1 bis 100 mbar betrieben wird;
C) die Sumpftemperatur der wenigstens einen Kolonnen auf Werte von 80 bis 200° C eingestellt wird;
D) die Sumpfflüssigkeitsmenge (das Volumen) Werte von 1 bis 30% des Gesamtvolumens an zu trennender Stoffmenge in der wenigstens einen Kolonne beträgt;
E) das Verhältnis von Sumpfabzugsmenge zu Zulaufmenge Werte von 0,01 bis 0,3, beträgt;
F) Packungskolonnen mit Packungen einer spezifischen Oberfläche von 100 bis 1000 m²/m³ verwendet werden; und
G) das Verhältnis von Rücklaufmenge zu Zulaufmenge einen Faktor von 0,5:1 bis 5:1 aufweist.

2. Verfahren nach Anspruch 1, wobei die Trennwandkolonne eine Trennwand (T) in Kolonnenlängsrichtung unter Ausbildung eines oberen (C) und unteren (D) gemeinsamen Kolonnenbereichs, eines Zulaufteils (A) und Entnahmeteils (B) aufweist, und die Zuführung des aufzutrennenden Gemischs im mittleren Teil des Zulaufbereichs sowie die Abführung des Produktes Ethoxyquin im mittleren Bereich des Entnahmeteils erfolgt.

3. Verfahren nach einem der vorigen Ansprüche, wobei nur eine Kolonne und diese in Form einer Trennwandkolonne eingesetzt wird.

4. Verfahren nach einem der Ansprüche 2 bis 4, wobei die Trennwandkolonne (TK) eine Trennwand (T) in Kolonnenlängsrichtung unter Ausbildung eines oberen gemeinsamen Kolonnenbereichs (1), einen unteren gemeinsamen Kolonnenbereichs (6), eines Zulaufteils (2, 4) mit Verstärkungsteil (2) und Abtriebsteil (4), sowie eines Entnahmeteils (3, 5) mit Verstärkungsteil (5) und Abtriebsteil (3) aufweist, wobei die Zuführung des aufzutrennenden Gemischs (Feed) im mittleren Bereich des Zulaufteils (2, 4), die Abführung der Hochsiederfraktion über Sumpf (Sumpfabzug C), die Abführung der Leichtsiederfraktion über Kopf (Kopfabzug A) und die Abführung der Mittelsiederfraktion aus dem mittleren Bereich des Entnahmeteils (3, 5) (Seitenabzug B) erfolgt.

5. Verfahren nach einem der vorigen Ansprüche, wobei die wenigstens eine Trennwandkolonne 30 bis 100 theoretische Trennstufen aufweist.

6. Verfahren nach einem der vorigen Ansprüche, wobei der obere gemeinsame Kolonnenbereich (1) der wenigstens einen Trennwandkolonne (TK) 5 bis 50 %, der Verstärkungsteil (2) des Zulaufteils (2, 4) der Kolonne 5 bis 50 %, der Abtriebsteil (4) des Zulaufteils der Kolonne 5 bis 50 %, der Verstärkungsteil (3) des Entnahmeteils (3, 5) der Kolonne 5 bis 50 %, der Abtriebsteil (5) des Entnahmeteils der Kolonne 5 bis 50 %, und der gemeinsame untere Bereich (6) der Kolonne 5 bis 50 %, der Gesamtzahl der theoretischen Trennstufen (nth) der Kolonne aufweist.

7. Verfahren nach einem der vorigen Ansprüche, wobei in der wenigstens einen Trennwandkolonne (TK) jeweils die Summe der Zahl der theoretischen Trennstufen der Teilbereiche (2) und (4) im Zulaufteil 80 bis 110 %, der Summe der Zahl der Trennstufen der Teilbereiche (3) und (5) im Entnahmeteil beträgt.

8. Verfahren nach einem der vorigen Ansprüche, wobei der Trennwandteil 40 bis 80%, der obere gemeinsame Kolonnenteil 5 bis 50%, der untere gemeinsame Kolonnenteil 5 bis 50% der Gesamtzahl der theoretischen Trennstufen der Trennwandkolonne aufweist.

9. Verfahren nach einem der vorigen Ansprüche, wobei der obere gemeinsame Kolonnenbereich (1) der wenigstens einen Trennwandkolonne (TK) 5 bis 50 %, der Verstärkungsteil (2) des Zulaufteils (2, 4) der Kolonne 5 bis 50 %, der Abtriebsteil (4) des Zulaufteils der Kolonne 5 bis 50 %, der Verstärkungsteil (3) des Entnahmeteils (3, 5) der Kolonne 5 bis 50 %, der Abtriebsteil (5) des Entnahmeteils der Kolonne 5 bis 50 %, und der gemeinsame untere Bereich (6) der Kolonne 5 bis 50 %, der Gesamtzahl der theoretischen Trennstufen (nth) der Kolonne aufweist, und wobei der durch die Trennwand (T) unterteilte Teilbereich der Trennwandkolonne(n) (TK) bestehend aus den Teilbereichen 2, 3, 4 und 5 oder Teilen davon mit geordneten Packungen oder Füllkörpern bestückt ist, und die Kolonne(n) wenigstens teilweise mit Füllkörpern oder geordneten Packungen, bevorzugt geordneten Packungen, besonders bevorzugt solchen aus Blech, gefüllt ist.

10. Verfahren nach einem der vorigen Ansprüche, wobei die Verweilzeit des Schwersieders im Sumpf weniger als 10 Stunden beträgt.

11. Verfahren zur Herstellung eines Ethoxyquin-enthaltenden Lebens- oder Futtermittels, **dadurch gekennzeichnet, dass** im Schritt
1) ein rohes Ethoxyquin-Produkt mindestens einem, bevorzugt nur einem, Schritt der kontinuierlichen Destillation unter Verwendung wenigstens einer Trennwandkolonne oder wenigstens zweier thermisch gekoppelten Kolonnen, wobei wenigstens eine der wenigstens zwei gekoppelten Kolonnen eine Trennwandkolonne ist, wobei
A) die wenigstens eine Kolonne eine Zahl an Trennstufen von 10 bis 100 aufweist;
B) die wenigstens eine Kolonne bei 1 bis 100 mbar betrieben wird;
C) die Sumpftemperatur der wenigstens einen Kolonnen auf Werte von 80 bis 200° C eingestellt wird;
D) die Sumpfflüssigkeitsmenge (das Volumen) Werte von 1 bis 30% des Gesamtvolumens an zu trennender Stoffmenge in der wenigstens einen Kolonne beträgt;
E) das Verhältnis von Sumpfabzugsmenge zu Zulaufmenge Werte von 0,01 bis 0,3, beträgt;
F) Packungskolonnen mit geordneten Packungen einer spezifischen Oberfläche von 100 bis 1000 m²/m³ verwendet werden; und
G) das Verhältnis von Rücklaufmenge zu Zulaufmenge einen Faktor von 0,5:1 bis 5:1 aufweist, und
H) die Verweilzeit des Schwersieders im Sumpf weniger als 5 Stunden beträgt, unterworfen wird;
wobei das Verfahren nach Schritt 1 bevorzugt keine weiteren nachfolgenden Aufreinigungsschritte des Ethoxyquins wie Adsorption, Wäsche, Kristallisation umfasst;
2) aus dem Verfahren von Schritt 1 ein Ethoxyquin mit einem p-Phenitidin-Gehalt von weniger als 50 ppm erhalten wird, und
3) das im Schritt 2 erhältliche Ethoxyquin in ein Lebens- oder Futtermittel eingebracht wird.

## Claims

1. A process for purifying ethoxyquin comprising p-phenetidine, comprising at least one step of continuous distillation using at least one dividing wall column or at least two thermally coupled columns, wherein at least one of the at least two coupled columns is a dividing wall column, wherein ethoxyquin is obtained comprising less than 100 ppm p-phenetidine, wherein
A) the at least one column has a number of separation stages of 10 to 100;
B) the at least one column is operated at 1 to 100 mbar;
C) the bottom temperature of the at least one column is set to values of 80 to 200°C;
D) the amount of bottoms liquid (the volume) is from 1 to 30% of the total volume of the amount of substance to be separated in the at least one column;
E) the ratio of bottoms takeoff amount to the amount of feed is from 0.01 to 0.3;
F) packed columns with structured packings of a specific surface area of 100 to 1000 m²/m³ are used; and
G) the ratio of amount of reflux to amount of feed has a factor of from 0.5:1 to 5:1.

2. The process according to claim 1, wherein the dividing wall column has a dividing wall (T) in the longitudinal direction of the column forming an upper (C) and lower (D) common column region, an inflow section (A) and an offtake section (B), and the mixture to be separated is fed to the middle part of the inflow region and the product ethoxyquin is removed from the middle region of the offtake section.

3. The process according to any of the preceding claims, wherein only one column, and said column in the form of a dividing wall column, is used.

4. The process according to any of claims 2 to 4, wherein the dividing wall column (TK) has a dividing wall (T) in the longitudinal direction of the column forming an upper common column region (1), a lower common column region (6), an inflow section (2, 4) with rectifying section (2) and stripping section (4), and an offtake section (3, 5) with rectifying section (5) and stripping section (3), wherein the mixture to be separated (feed) is fed to the middle region of the inflow section (2, 4), the high boiler fraction is removed via the bottom (bottom takeoff C), the low boiler fraction is removed via the overhead (top takeoff A) and the intermediate boiler fraction is removed from the middle region of the offtake section (3, 5) (side takeoff B).

5. The process according to any of the preceding claims, wherein the at least one dividing wall column has 30 to 100 theoretical plates.

6. The process according to any of the preceding claims, wherein the upper common column region (1) of the at least one dividing wall column (TK) has 5 to 50%, the rectifying section (2) of the inflow section (2, 4) of the column has 5 to 50%, the stripping section (4) of the inflow section of the column has 5 to 50%, the rectifying section (3) of the offtake section (3, 5) of the column has 5 to 50%, the stripping section (5) of the offtake section of the column has 5 to 50%, and the common lower region (6) of the column has 5 to 50%, of the total number of theoretical plates (nth) of the column.

7. The process according to any of the preceding claims, wherein in the at least one dividing wall column (TK), in each case the sum of the number of theoretical plates of the subregions (2) and (4) in the inflow section is 80 to 110% of the total of the number of plates of the subsections (3) and (5) in the offtake section.

8. The process according to any of the preceding claims, wherein the dividing wall section has 40 to 80%, the upper common column section has 5 to 50%, the lower common column section has 5 to 50%, of the total number of theoretical plates of the dividing wall column.

9. The process according to any of the preceding claims, wherein the upper common column region (1) of the at least one dividing wall column (TK) has 5 to 50%, the rectifying section (2) of the inflow section (2, 4) of the column has 5 to 50%, the stripping section (4) of the inflow section of the column has 5 to 50%, the rectifying section (3) of the offtake section (3, 5) of the column has 5 to 50%, the stripping section (5) of the offtake section of the column has 5 to 50%, and the common lower region (6) of the column has 5 to 50%, of the total number of theoretical plates (nth) of the column, and wherein the subsection of the dividing wall column(s) (TK) divided by the dividing wall (T) consisting of subsections 2, 3, 4 and 5 or parts thereof is loaded with ordered packings or random packings, and the column(s) is filled at least partially with random packings or ordered packings, preferably ordered packings, particularly preferably those composed of sheet metal.

10. The process according to any of the preceding claims, wherein the residence time of the high boiler in the bottoms is less than 10 hours.

11. A process for producing an ethoyquin-containing foodstuff or feedstuff, wherein in step
1) a crude ethoxyquin product is subjected to at least one, preferably only one, step of continuous distillation using at least one dividing wall column or at least two thermally coupled columns, wherein at least one of the at least two coupled columns is a dividing wall column, wherein
A) the at least one column has a number of separation stages of 10 to 100;
B) the at least one column is operated at 1 to 100 mbar;
C) the bottom temperature of the at least one column is set to values of 80 to 200°C;
D) the amount of bottoms liquid (the volume) is from 1 to 30% of the total volume of the amount of substance to be separated in the at least one column;
E) the ratio of bottoms takeoff amount to the amount of feed is from 0.01 to 0.3;
F) packed columns with ordered packings of a specific surface area of 100 to 1000 m²/m³ are used; and
G) the ratio of amount of reflux to amount of feed has a factor of from 0.5:1 to 5:1, and
H) the residence time of the high boiler in the bottoms is less than 5 hours;
wherein the process according to step 1 preferably does not comprise any subsequent ethoxyquin purification steps such as adsorption, washing, crystallization;
2) from the process of step 1, ethoxyquin having a p-phenetidine content of less than 50 ppm is obtained, and
3) the ethoxyquin obtainable in step 2 is incorporated in a foodstuff or feedstuff.

## Revendications

1. Procédé pour la purification d'éthoxyquine contenant de la p-phénitidine, comprenant au moins une étape de distillation continue à l'aide d'au moins une colonne à paroi de séparation ou d'au moins deux colonnes thermiquement couplées, au moins l'une des dites au moins deux colonnes couplées étant une colonne à paroi de séparation, une éthoxyquine comprenant moins de 100 ppm de p-phénitidine étant obtenue, où
A) ladite au moins une colonne présente un nombre de plateaux de séparation de 10 à 100 ;
B) ladite au moins une colonne fonctionne à 1 jusqu'à 100 mbars ;
C) la température du fond de ladite au moins une colonne est réglée à des valeurs de 80 à 200°C ;
D) la quantité de liquide du fond (le volume) représente des valeurs de 1 à 30% du volume total de la quantité de substance à séparer dans ladite au moins une colonne ;
E) le rapport de la quantité soutirée du fond à la quantité alimentée présente des valeurs de 0,01 à 0,3 ;
F) des colonnes garnies présentant des garnissages d'une surface spécifique de 100 à 1000 m²/m³ sont utilisées ; et
G) le rapport de la quantité de reflux à la quantité alimentée présente un facteur de 0,5:1 à 5:1.

2. Procédé selon la revendication 1, la colonne à paroi de séparation présentant une paroi de séparation (T) dans la direction longitudinale de la colonne avec formation d'une zone commune supérieure de colonne (C) et d'une zone commune inférieure de colonne (D), d'une zone d'alimentation (A) et d'une zone de prélèvement (B) et l'alimentation du mélange à séparer ayant lieu dans la partie centrale de la partie d'alimentation et l'évacuation du produit éthoxyquine ayant lieu dans la partie centrale de la partie de prélèvement.

3. Procédé selon l'une quelconque des revendications précédentes, une seule colonne étant utilisée et celle-ci étant utilisée sous forme d'une colonne à paroi de séparation.

4. Procédé selon l'une quelconque des revendications 2 à 4, la colonne à paroi de séparation (TK) présentant une paroi de séparation (T) dans la direction longitudinale de la colonne avec formation d'une zone commune supérieure de colonne (1), d'une zone commune inférieure de colonne (6), d'une partie d'alimentation (2, 4) présentant une partie de concentration (2) et une partie de rectification (4), ainsi que d'une partie de prélèvement (3, 5) présentant une partie de concentration (5) et une partie de rectification (3), l'introduction du mélange à séparer (Feed) ayant lieu dans la zone centrale de la partie d'alimentation (2, 4), l'évacuation de la fraction à point d'ébullition élevé ayant lieu via le fond (soutirage du fond C), l'évacuation de la fraction à bas point d'ébullition ayant lieu via la tête (soutirage de tête A) et l'évacuation de la fraction à point d'ébullition moyen ayant lieu dans la zone centrale de la partie de prélèvement (3, 5) (soutirage latéral B).

5. Procédé selon l'une quelconque des revendications précédentes, ladite au moins une colonne à paroi de séparation présentant 30 à 100 plateaux théoriques de séparation.

6. Procédé selon l'une quelconque des revendications précédentes, la zone commune supérieure de colonne (1) de ladite au moins une colonne à paroi de séparation (TK) présentant 5 à 50%, la partie de concentration (2) de la partie d'alimentation (2, 4) de la colonne présentant 5 à 50%, la partie de rectification (4) de la partie d'alimentation de la colonne présentant 5 à 50%, la partie de concentration (3) de la partie de prélèvement (3, 5) de la colonne présentant 5 à 50%, la partie de rectification (5) de la partie de prélèvement de la colonne présentant 5 à 50% et la zone commune inférieure de colonne (6) présentant 5 à 50% du nombre total de plateaux théoriques de séparation (nth) de la colonne.

7. Procédé selon l'une quelconque des revendications précédentes, où, dans ladite au moins une colonne à paroi de séparation (TK), la somme du nombre de plateaux théoriques de séparation des zones partielles (2) et (4) dans la partie d'alimentation représentant 80 à 110% de la somme du nombre de plateaux de séparation des zones partielles (3) et (5) dans la partie de prélèvement.

8. Procédé selon l'une quelconque des revendications précédentes, la partie de paroi de séparation présentant 40 à 80%, la partie commune supérieure de colonne présentant 5 à 50%, la partie commune inférieure de colonne présentant 5 à 50% du nombre total des plateaux théoriques de séparation de la colonne à paroi de séparation.

9. Procédé selon l'une quelconque des revendications précédentes, la zone commune supérieure de colonne (1) de ladite au moins une colonne à paroi de séparation (TK) présentant 5 à 50%, la partie de concentration (2) de la partie d'alimentation (2, 4) de la colonne présentant 5 à 50%, la partie de rectification (4) de la partie d'alimentation de la colonne présentant 5 à 50%, la partie de concentration (3) de la partie de prélèvement (3, 5) de la colonne présentant 5 à 50%, la partie de rectification (5) de la partie de prélèvement de la colonne présentant 5 à 50% et la zone commune inférieure de colonne (6) présentant 5 à 50% du nombre total de plateaux théoriques de séparation (nth) de la colonne et la zone partielle de la/des colonne(s) à paroi de séparation (TK) divisée par la paroi de séparation (T), constituée par les zones partielles 2, 3, 4 et 5 ou des parties de celles-ci, étant pourvue de garnissages ou de corps de remplissage ordonnés et la/les colonne(s) étant remplie(s) au moins partiellement de corps de remplissage ou de garnissages ordonnés, de préférence de garnissages ordonnés, de manière particulièrement préférée de garnissages ordonnés en tôle.

10. Procédé selon l'une quelconque des revendications précédentes, la durée de séjour de la fraction lourde dans le fond étant inférieure à 10 heures.

11. Procédé pour la préparation d'une denrée alimentaire ou d'un aliment pour animaux, **caractérisé en ce que** dans l'étape
1) un produit d'éthoxyquine brut est soumis à au moins une étape, de préférence une seule étape, de distillation continue à l'aide d'au moins une colonne à paroi de séparation ou d'au moins deux colonnes thermiquement couplées, au moins l'une desdites au moins deux colonnes couplées étant une colonne à paroi de séparation, où
A) ladite au moins une colonne présente un nombre d'étages de séparation de 10 à 100 ;
B) ladite au moins une colonne fonctionne à 1 jusqu'à 100 mbars ;
C) la température du fond de ladite au moins une colonne est réglée à des valeurs de 80 à 200°C ;
D) la quantité de liquide du fond (le volume) représente des valeurs de 1 à 30% du volume total de la quantité de substance à séparer dans ladite au moins une colonne ;
E) le rapport de la quantité soutirée du fond à la quantité alimentée présente des valeurs de 0,01 à 0,3 ;
F) des colonnes garnies présentant des garnissages ordonnés d'une surface spécifique de 100 à 1000 m²/m³ sont utilisées ; et
G) le rapport de la quantité de reflux à la quantité alimentée présente un facteur de 0,5:1 à 5:1 et
H) la durée de séjour de la fraction lourde dans le fond est inférieure à 5 heures ;
le procédé, après l'étape 1, ne comprenant de préférence pas d'autres étapes de purification consécutives de l'éthoxyquine, telles que l'adsorption, le lavage, la cristallisation ;
2) une éthoxyquine présentant une teneur en p-phénitidine inférieure à 50 ppm est obtenue à partir du procédé de l'étape 1 et
3) l'éthoxyquine pouvant être obtenue dans l'étape 2 est introduite dans une denrée alimentaire ou un aliment pour animaux.
